(19) Europäisches Patentamt
European Patent Office
Office européen des brevets

(11) Publication number : **0 153 834 B2**

(12) **NEW EUROPEAN PATENT SPECIFICATION**

(45) Date of publication of the new patent specification : **09.06.93 Bulletin 93/23**

(51) Int. Cl.$^5$ : **C07C 53/08,** C07C 51/12, B01J 31/30

(21) Application number : **85300999.1**

(22) Date of filing : **14.02.85**

(54) **Carbonylation process improvement.**

The file contains technical information submitted after the application was filed and not included in this specification

(30) Priority : **16.02.84 GB 8404136**

(43) Date of publication of application : **04.09.85 Bulletin 85/36**

(45) Publication of the grant of the patent : **04.01.89 Bulletin 89/01**

(45) Mention of the opposition decision : **09.06.93 Bulletin 93/23**

(84) Designated Contracting States : **AT BE DE FR GB IT LU NL SE**

(56) References cited :
**EP-A- 0 008 396**
**EP-A- 0 042 633**
**EP-A- 0 055 618**

(56) References cited :
**EP-A- 0 079 461**
**DE-A- 2 450 965**
**DE-A- 2 610 036**
**FR-A- 2 303 597**
**GB-A- 1 326 014**
**US-A- 3 769 329**

(73) Proprietor : **BP Chemicals Limited**
**Belgrave House 76 Buckingham Palace Road**
**London, SW1W 0SU (GB)**

(72) Inventor : **Ray, David John Mathieson**
**BP Chemicals Limited Saltend, Hedon**
**Hull, JU12 8DS (GB)**
Inventor : **Stringer, Allan Joseph**
**BP Chemicals Limited Saltend, Hedon**
**Hull, HU12 8DS (GB)**

(74) Representative : **Fawcett, Richard Fennelly et al**
**BP INTERNATIONAL LIMITED Patents Division Chertsey Road**
**Sunbury-on-Thames Middlesex, TW16 7LN (GB)**

EP 0 153 834 B2

## Description

The present invention relates to improvements in carbonylation processes. More particularly, the invention relates to improvements in carbonylation processes which arise from stabilising the compound or compounds which act as catalysts for such processes.

Carbonylation processes in which small organic molecules, for example nonpolar molecules such as alkenes or alkynes or polar molecules such as alcohols, esters, or ethers, are reacted with carbon monoxide in the liquid phase and in the presence of a transition metal catalyst are known and in some cases used commercially.

In particular, one successful class of carbonylation processes is that in which the active catalyst is derived from a compound of the Group VIII metal rhodium, used in conjunction with a halide promoter.

When compared with other transition metal complexes, such as cobalt carbonyl, nickel carbonyl or iron carbonyl, which are often regarded as 'classical' carbonylation catalysts, rhodium catalysts can be seen to have two advantages. First the rhodium catalysts are more reactive which means that for a given amount of feedstock the rate of reaction is much greater. This leads to two possible desirable options; either the throughput of feedstock can be increased or, as rate of reaction is related to temperature, the process can be operated at lower temperature. Either option can lead to substantial reductions in overall process costs.

A second desirable attribute which rhodium catalysts possess is that, relative to 'classical' carbonylation catalysts, they exhibit superior thermal stability. This is particularly important from a process viewpoint since, in general, at a given temperature the only way to overcome problems of catalyst deactivation is to work at elevated carbon monoxide pressure. Thus, carbonylation processes which formerly used classical catalysts always involved working at high temperatures, frequently in excess of 150 bars. Rhodium catalysts however allow similar reactions to be carried out, with equivalent or superior productivities, at pressures of less than 75 bars. By working at lower pressures many of the problems and costs associated with the design and use of high pressure processing equipment are obviated.

Rhodium catalysts have only one major drawback over the classical carbonylation catalysts. Because rhodium is a relatively rare metal which is difficult to purify, its cost per unit weight is substantially in excess of more common metals such as cobalt, nickel and iron. As a consequence, rhodium carbonylation catalysts are extremely expensive and it is desirable that quantitiative catalyst recovery from the reactor and product stream is, as far as possible, effected.

The most effective method of ensuring that catalyst losses are reduced is to add a stabiliser which prevents precipitation of the catalyst from the liquid streams. This is particularly important in the product stream since, at some point after reaction the carbon monoxide overpressure is reduced and hence the probability of small amounts of catalyst precipitating from solution is increased. However, it will be appreciated by the skilled chemist that the size of a commercial plant is such that small individual catalyst losses in, for example, the reactor, transfer lines and recycle lines can amount to substantial total losses.

Additives which stabilise rhodium catalysts during liquid phase carbonylation of nonpolar olefinic feedstocks have been disclosed. For example, from US 3,818,060 it is known that organic derivatives of pentavalent phosphorus, arsenic, antimony, nitrogen or bismouth may be used as stabilisers during the liquid phase carbonylation of ethylenically unsaturated compounds. Furthermore, from US 3,579,552 it is known that inter alia phosphines, amine and trihalostannates can also be used to produce soluble rhodium coordination complexes for the same reaction.

With regard to carbonylation reactions which involve the use of polar feedstocks, e.g. alcohols, esters and ethers, and a rhodium/halide catalyst European patent application 0 055 618A discloses four classes of stabiliser

(1) N,N,N',N'-tetramethyl-o-phenylendiamine and 2,3'dipyridyl.

(2) a substituted diphosphine having the formula

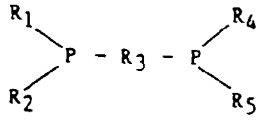

wherein $R_1$, $R_2$, $R_4$ and $R_5$ are alkyl or substituted alkyl groups and $R_3$ a polymethylene group

(3) a dibasic or polybasic acid having the formula

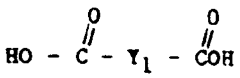 or 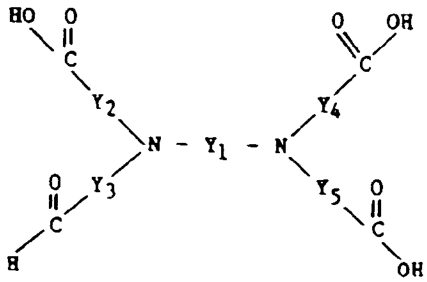

wherein $Y_1$ is of the formula $(CX_1X_2)_m$ in which m is 2 to 10 and wherein $Y_2$, $Y_3$, $Y_4$ and $Y_5$ are independently selected from hydrogen, a halogen, lower alkyl, aryl, hydroxyl, carboxyl amino, hydroxy-substituted alkyl and carboxysubstituted alkyl or

(4) a compound of germanium antimony, tin or an alkali metal all of which reduce the loss of rhodium catalyst from solution in those parts of the carbonylation plant where the catalyst is exposed to conditions which are deficient in carbon monoxide.

It has now been discovered that when polar feedstocks such as those disclosed in European patent application 0 055 618A are used, a further class of compound may be used to stabilise the rhodium catalyst system and prevent catalyst loss by precipitation. Thus, in this invention, precipitation of rhodium in rhodium/halide catalyst systems generally under carbon monoxide deficient conditions is prevented or retarded by the addition to the system of a stabilising compound which is an imidazole.

Accordingly, the present invention comprises a process for preparing acetic acid by carbonylation of a methanol feedstock which comprises (a) reacting the feedstock with carbon monoxide at superatmospheric pressure and elevated temperature in a liquid reaction medium containing a rhodium catalyst, an iodide or bromide components, an imidazole stabilising compound, water and acetic acid, (b) transferring the product of step (a) to a zone in which the carbon monoxide overpressure is reduced and acetic acid is removed by distillation and (c) thereafter recycling the rhodium catalyst and imidazole stabilising compound to step (a).

In another embodiment a process for reducing the loss of rhodium through precipitation under the carbon monoxide deficient conditions found in plant producing acetic acid by the above process is provided. The process comprises keeping the rhodium in contact with the stabilising compound during and after the point where the carbon monoxide overpressure is reduced.

The feedstocks which may be carbonylated in the process contemplated comprise methanol, methyl acetate or a mixture thereof. The feedstocks may optionally contain water (as shown, for example, in Example 1).

Any soluble rhodium containing catalyst useful in the carbonylation of alcohols, esters or ethers may be used herein. The source of rhodium may be, for example, a simple inorganic salt such as rhodium chloride, bromide, iodide, or nitrate; a carbonyl or organometallic complex of rhodium, or a coordination complex. Finely divided rhodium metal which becomes solubilised in the reaction medium may also be used.

When the iodide or bromide component used in conjunction with the catalyst is an iodide, it can be added as elemental iodine, hydrogen iodide, an iodide salt, for example sodium iodide, or an organic source of iodide such as an alkyl or aryl iodide. A preferred source of the iodide component is methyl iodide. It is possible to supply part of the iodide with the rhodium by using, for example, a compound such as rhodium triiodide. The concentration of iodide is such as to produce a rhodium to iodide ratio of at least 1:4 preferably between 1:10 and 1:1000. When a bromide component is used, it may be added in a corresponding way and combinations of iodide and bromide can be employed.

The imidazole used as the stabiliser will have the general formula

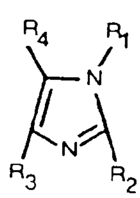

in which $R_1$, $R_2$, $R_3$ and $R_4$ are each independently hydrogen, alkyl, aryl, cycloalkyl or alkaryl hydrocarbyl radicals. A preferred imidazole is N-methylimidazole.

The stabilising compound is present in amounts such that the molar ratio of stabilising compound to rhodium component is at least 0.5: 1 and preferably in the range 0.5:1 to $10^5$:1.

The carbonylation reaction as described herein is carried out by contacting a solution of the catalyst system in the polar feedstock with carbon monoxide in an appropriate reactor. The concentration of the soluble rhodium component will in general be such as to constitute between 10 ppm and 3000 ppm of the reaction mixture.

As mentioned previously, the reaction is carried out under superatmospheric pressure and at elevated temperature. Although the optimum conditions will depend on the particular feedstock used, the reaction is generally carried out at a pressure of greater than 10 bars, preferably 10 to 100 bars and at a temperature in the range of 100 to 250°C. For the preferred feedstocks mentioned herein, the optimum temperature and pressure ranges will vary somewhat. However, the ranges of such optimum temperatures and pressure for a given feedstock will be familiar to those skilled in the art of carbonylation.

It is preferable that the carbon monoxide used in this invention is as pure as possible. However, a certain amount of inert diluent gases such as nitrogen or gases which are often co-produced with carbon monoxide, such as hydrogen, may be present. If hydrogen is present it should be at level less than 50 mole% of the total gas stream.

The stabilising influence of the imidazoles described herein will now be illustrated by the following Examples. However, these Examples should not be construed as limiting the scope of this invention which includes equivalent embodiments, modifications and variations.

Example 1

An autoclave was charged with methanol (78.9 g), methyl iodide (49.3 g), acetic acid (178.5 g) and water (51.3 g). Rhodium trichloride was added at a level such that the initial rhodium concentration in solution was 340 ppm. Enough 1,2-ethanedithiol was added to give a 1,2-ethanedithiol to rhodium molar ratio of 2.5:1. The autoclave was then sealed and the temperature and carbon monoxide overpressure adjusted to 185°C and 400psig (ca 27 bars) respectively. Under these conditions the liquid phase carbonylation of methanol to acetic acid took place. After 45 minutes the rhodium content of the product solution was determined by atomic absorption spectroscopy. The results showed a concentration of 308 ppm indicating that 93% of the rhodium still remained in solution. The weight of the product solution was 368.7 g.

Comparative Test A

An autoclave was charged with methanol (78.3 g), methyl iodide (47.7 g), acetic acid (177.1 g) and water (50.4 g). Rhodium trichloride was added at a level such that the initial rhodium concentration in solution was 340 ppm, but no stabiliser was added. After 45 minutes under conditions identical to those used in Example 1, analysis of the product solution by atomic absorption spectroscopy showed a rhodium concentration of 315 ppm indicating that in this case only 87% of the rhodium still remained in solution. The weight of the product solution was 333 g.

Example 2

Methanol (625 g/hr) was fed to a reactor, at 175°C and 27 bars pressure, which contained a rhodium/iodide catalyst and as stabiliser N-methylimidazole. The molar ratio of rhodium to iodide was 1:185 and the molar ratio of stabiliser to rhodium was 80:1. The catalyst and stabiliser were separated from the acetic acid, containing product stream, by distillation, downstream of the reactor and subsequently recycled. Loss of rhodium from the liquid phase was followed by analysing samples of the product stream for rhodium at various times. The change in rhodium in solution over 50 hours, as measured by atomic absorption, is shown in the Table.

| TIME ON STREAM (HRS) | CONCENTRATION OF RHODIUM IN LIQUID PHASE (PPM) |
|---|---|
| 0 | 540 |
| 8 | 542 |
| 18.7 | 547 |
| 49.5 | 491 |

Example 2 shows that the addition of N-methylimidazole as stabiliser reduces the loss of rhodium from

the solution. Under equivalent conditions, in the absence of stabilisers, rhodium losses from solution approach 100 ppm/24 hours.

## Claims

1. A process for preparing acetic acid by carbonylation of a methanol feedstock which comprises (a) reacting the feedstock with carbon monoxide at superatmospheric pressure and elevated temperature in a liquid reaction medium containing a rhodium catalyst, an iodide or bromide component, an imidazole stabilising compound, water and acetic acid, (b) transferring the product of step (a) to a zone in which the carbon monoxide overpressure is reduced and acetic acid is removed by distillation and (c) thereafter recycling the rhodium catalyst and imidazole stabilising compound to step (a).

2. A process as claimed in Claim 1 wherein the iodide or bromide component is an alkyl or aryl iodide.

3. A process as claimed in Claim 2 wherein the alkyl iodide component is methyl iodide.

4. A process as claimed in Claim 1 wherein the iodide or bromide component is an iodide salt.

5. A process as claimed in Claim 4 wherein the iodide salt comprises sodium iodide.

6. A process as claimed in any of the preceding claims wherein the molar ratio of stabilising compound to rhodium component is in the range 0.5:1 to $10^5$:1.

7. A process as claimed in any of the preceding claims wherein the imidazole is N-methylimidazole.

8. A process as claimed in any of the preceding claims wherein the methanol feedstock contains water.

9. A process as claimed in any of the preceding claims wherein the rhodium to iodide or bromide component molar ratio is between 1:10 and 1:1000.

10. A process for preparing acetic cacid by carbonylation of a methanol feedstock which comprises (a) reacting the feedstock with carbon monoxide at a pressure in the range 10 to 100 bars and at a temperature in the range 100 to 250°C in a liquid reaction medium containing a rhodium catalyst, methyl iodide, N-methylimidazole, water and acetic acid, (b) transferring the product of step (a) to a zone in which the carbon monoxide overpressure is reduced and acetic acid is separated by distillation and (c) thereafter recycling the rhodium catalyst and N-methylimidazole to step (a).

11. A process for reducing the loss of rhodium through precipitation under the carbon monoxide deficient conditions found in plant producing acetic acid by a process as claimed in either Claim 1 or Claim 10 which comprises keeping the rhodium and the imidazole stabilising compound together during and after the point in the plant where the carbon monoxide overpressure is reduced.

12. A process as claimed in Claim 11 wherein the rhodium and imidazole are kept together during passage through the transfer lines and recycle lines of the plant.

## Patentansprüche

1. Verfahren zur Herstellung von Essigsäure durch Carbonylierung eines Methanolausgangsmaterials, das umfaßt (a) Umsetzen des Ausgangsmaterials mit Kohlenmonoxid bei überatmosphärischem Druck und erhöhter Temperatur in einem flüssigen Reaktionsmedium, enthaltend einen Rhodiumkatalysator, eine Iodid- oder Bromidkomponente, eine stabilisierende Imidazolverbindung, Wasser und Essigsäure, (b) Übertragen des Produkts aus Schritt (a) in einen Bereich, wo der Kohlenmonoxidüberdruck reduziert und die Essigsäure durch Destillation entfernt wird und (c) danach Zurückführen des Rhodiumkatalysators und der stabilisierenden Imidazolverbindung zu Schritt (a).

2. Verfahren nach Anspruch 1, worin die Iodid- oder Bromidkomponente ein Alkyl- oder Aryliodid ist.

3. Verfahren nach Anspruch 2, worin die Alkyliodidkomponente Methyliodid ist.

4. Verfahren nach Anspruch 1, worin die Iodid- oder Bromidkomponente ein Iodidsalz ist.

5. Verfahren nach Anspruch 4, worin das Iodidsalz Natrium-iodid umfaßt.

6. Verfahren nach einem der vorstehenden Ansprüche, worin das Molverhältnis von stabilisierender Verbindung zu Rhodiumkomponente im Bereich von 0,5:1 bis $10^5$:1 liegt.

7. Verfahren nach einem der vorstehenden Ansprüche, worin das Imidazol N-Methylimidazol ist.

8. Verfahren nach einem der vorstehenden Ansprüche, worin das Methanolausgangsmaterial Wasser enthält.

9. Verfahren nach einem der vorstehenden Ansprüche, worin das Molverhältnis von Rhodium zu der Iodid- oder Bromidkomponente zwischen 1:10 und 1:1000 liegt.

10. Verfahren zur Herstellung von Essigsäure durch Carbonylierung eines Methanolausgangmaterials, das umfaßt (a) Umsetzen des Ausgangmaterials mit Kohlenmonoxid bei einem Druck im Bereich von 10 bis 100 bar und bei einer Temperatur im Bereich von 100 bis 250°C in einem flüssigen Reaktionsmedium, das einen Rhodiumkatalysator, Methyliodid, N-Methylimidazol, Wasser und Essigsäure enthält, (b) Übertragen des Produkts aus Schritt (a) in einen Bereich, wo der Kohlenmonoxidüberdruck reduziert und die Essigsäure durch Destillation entfernt wird und (c) danach Zurückführen des Rhodiumkatalysators und des N-Methylimidazols zu Schritt (a).

11. Verfahren zur Verminderung der Rhodiumverluste durch Ausfällung unter Kohlenmonoxidmangelbedingungen, die in Anlagen, die Essigsäure durch ein Verfahren nach Anspruch 1 oder 10 herstellen, auftreten, welches umfaßt, daß das Rhodium und die Imidazol-Stabilisierungsverbindung während und nach der Stelle in der Anlage, wo der Kohlenmonoxid-Überdruck vermindert ist, zusammengehalten werden.

12. Verfahren nach Anspruch 11, worin das Rhodium und das Imidazol während des Durchtritts durch die Transferleitungen und Rücklaufleitungen der Anlage zusammengehalten werden.

## Revendications

1. Procédé pour préparer de l'acide acétique par carbonylation d'une charge méthanolique d'alimentation, qui comprend (a) la réaction de la charge d'alimentation avec du monoxyde de carbone, sous une pression supérieure à la pression atmosphérique et à température élevée, dans un milieu liquide de réaction contenant un catalyseur au rhodium, un constituant iodure ou bromure, un composé stabilisant qui est un imidazole, ainsi que de l'eau et de l'acide acétique, (b) le transfert du produit de l'étape (a) vers une zone dans laquelle la surpression du monoxyde de carbone est diminuée et l'acide acétique est enlevé par distillation, et (c) puis le recyclage, vers l'étape (a), du catalyseur au rhodium et de l'imidazole, composé stabilisant.

2. Procédé tel que revendiqué à la revendication 1, dans lequel le constituant iodure ou bromure est un iodure d'alkyle ou d'aryle.

3. Procédé tel que revendiqué à la revendication 2, dans lequel le constituant iodure ou bromure est l'iodure de méthyle.

4. Procédé tel que revendiqué à la revendication 1, dans lequel le constituant iodure ou bromure est un iodure salin.

5. Procédé tel que revendiqué à la revendication 4, dans lequel l'iodure salin est ou comprend de l'iodure de sodium.

6. Procédé tel que revendiqué dans l'une quelconque des revendications précédentes, dans lequel le rapport molaire du composé stabilisant au constituant rhodium se situe entre 0,5:1 et $10^5$:1.

7. Procédé tel que revendiqué dans l'une quelconque des revendications précédentes, dans lequel l'imidazole est le N-méthylimidazole.

**8.** Procédé tel que revendiqué dans l'une quelconque des revendications précédentes, dans lequel la charge méthanolique d'alimentation contient de l'eau.

**9.** Procédé tel que revendiqué dans l'une quelconque des revendications précédentes, dans lequel le rapport molaire du rhodium au constituant iodure ou bromure se situe entre 1:10 et 1:1000.

**10.** Procédé pour préparer de l'acide acétique par carbonylation d'une charge méthanolique d'alimentation, qui comprend (a) la réaction de la charge d'alimentation avec du monoxyde de carbone à une pression comprise entre 10 et 100 bars et à une température comprise entre 100 et 250 °C dans un milieu liquide de réaction contenant un catalyseur au rhodium, de l'iodure de méthyle, du N-méthylimidazole, de l'eau et de l'acide acétique, (b) le transfert du produit de l'étape (a) vers une zone dans laquelle la surpression du monoxyde de carbone est diminuée et l'acide acétique est séparé par distillation, et (c), ensuite, le recyclage vers l'étape (a) du catalyseur au rhodium et du N-méthylimidazole.

**11.** Procédé pour diminuer la perte de rhodium par précipitation, dans les conditions déficientes en monoxyde de carbone que l'on trouve dans une installation produisant de l'acide acétique par un procédé tel que revendiqué dans l'une des revendications 1 ou 10, qui comprend le maintien du rhodium et de l'imidazole, composé stabilisant, ensemble pendant et après l'instant ou le point dans l'installation où la surpression en monoxyde de carbone est diminuée.

**12.** Procédé tel que revendiqué à la revendication 11, dans lequel on maintient ensemble le rhodium et l'imidazole pendant leur passage par les conduits de transfert et les conduits de recyclage faisant partie de l'installation.